# EUROPÄISCHE PATENTANMELDUNG

(11) **EP 0 779 070 A1**
(43) Veröffentlichungstag der Anmeldung: **18.06.1997**
(21) Anmeldenummer: 96119793.6
(22) Anmeldetag: 10.12.1996
(51) Int. Cl.: A61K 7/50, B01F 17/00

(54) **Alkylpolyglycosid enthaltende Ölemulsion**

(30) Priorität: 12.12.1995 DE 19546416
(71) Anmelder: BASF AKTIENGESELLSCHAFT, 67056 Ludwigshafen (DE)
(72) Erfinder: Oppenländer, Knut, Dr., 67061 Ludwigshafen (DE); Dralle-Voss, Gabriele, Dr., 64297 Darmstadt (DE); Oetter, Günter, Dr., 67227 Frankenthal (DE); Wolf, Gerhard, Dr., 68775 Ketsch (DE); Wekel, Hans-Ulrich, Dr., 67158 Ellerstadt (DE)
(74) Vertreter: Isenbruck, Günter, Dr.

(57) **Zusammenfassung**

Die Erfindung betrifft Ölemulsionen, umfassend
(a) mindestens ein Alkylpolyglycosid der allgemeinen Formel

   R―O―(Z)ₙ―H

   in der der Rest R ein linearer oder verzweigter gesättigter oder ungesättigter Alkylrest mit 8 bis 16, vorzugsweise 8 bis 12 C-Atomen ist, der Rest ―(Z)ₙ―H ein Polyglycosylrest ist, Z ein Glycosylrest ist und n im Mittel einen Wert von 1 bis 5 aufweist,
(b) mindestens ein Öl,
(c) Wasser,
wobei das Gewichtsverhältnis von Komponente (a) zu Komponente (b) 1,5:1 bis 15:1, vorzugsweise 2,5:1 bis 10:1 beträgt,
sowie die Verwendung von C₈/C₁₀-Alkylpolyglycosiden als Emulgiermittel für Öle.

## Beschreibung

Die Erfindung betrifft Ölemulsionen in Wasser oder Wasser/Alkohol-Gemischen, vorzugsweise von etherischen Ölen, die in der Kosmetik oder Pharmazeutik eingesetzt werden können.

Aus der DE-A1-41 10 506 sind Emulgatoren zur Herstellung von in der Kosmetik oder Medizin verwendbaren Öl-in-Wasser-Emulsionen etherischer Öle bekannt. Als Emulgatoren werden C₁₀/C₁₂- und C₁₂/C₁₄-Alkylpolyglucoside verwendet. Das Verhältnis von Alkylpolyglucosid zu Öl beträgt maximal 1:4.

In der DE-A1-40 33 928 sind Öl-in-Wasser-Emulsionen beschrieben, die Alkylpolyglycoside enthalten. Es sind C₁₄-, C₁₆-, und C₁₈-Alkylglucoside beschrieben. Das Gewichtsverhältnis von Alkylpolyglucosid zu Öl beträgt maximal 1:3. Die Öl-Emulsionen enthalten ferner als Kristallisationsinhibitor ein Fettsäurepartialglycerid.

In der EP-A1-0 418 479 sind Emulgatoren zur Herstellung von lagerstabilen, wäßrigen Polysiloxan- bzw. Polysiloxan-Paraffinöl-Emulsionen beschrieben. Es werden C₁₀/C₁₂-, C₁₂/C₁₃- und C₁₂/C₁₄-Alkylpolyglycoside verwendet, wobei das Gewichtsverhältnis von Öl zu Alkylpolyglycosid maximal 4:1 beträgt.

Eine Aufgabe der vorliegenden Erfindung ist die Bereitstellung von Ölemulsionen, die toxikologisch unbedenklich sind.

Eine weitere Aufgabe der vorliegenden Erfindung ist die Bereitstellung von Ölemulsionen, die klar und stabil sind.

Eine weitere Aufgabe der vorliegenden Erfindung ist die Bereitstellung von Ölemulsionen, die ein gutes Schaumvermögen aufweisen.

Diese Aufgaben werden erfindungsgemäß gelöst durch eine Ölemulsion, wie sie in den Patentansprüchen beschrieben ist sowie durch Verwendung der erfindungsgemäßen Alkylpolyglycoside.

Erfindungsgemäß wurde gefunden, daß bei Verwendung von Alkylpolyglycosiden als Emulgatoren Ölemulsionen aus Wasser-in-Öl-Gemischen erhalten werden, die ein klares Aussehen, eine große Stabilität und ein hohes Schaumvermögen aufweisen.

Gegebenenfalls können die erfindungsgemäß eingesetzten Alkylpolyglycoside mit weiteren Coemulgatoren verwendet werden, die nicht-ionische, kationische oder anionische Tenside sind. Die Alkylpolyglycoside sind bekannt für ihre Hautfreundlichkeit und gute Haut- und Schleimhautverträglichkeit. Sie sind toxikologisch unbedenklich.

### Alkylpolyglycoside

Unter dem Begriff "Alkylpolyglycoside" werden die Reaktionsprodukte aus Zuckern und aliphatischen Alkoholen verstanden. Die erfindungungsgemäß eingesetzten Alkylpolyglycoside können allgemein beschrieben werden durch die Formel R-O-(Z)ₙ-H, wobei der Rest R einen linearen oder verzweigten, gesättigten oder ungesättigten C₈₋₁₆-Alkylrest, vorzugsweise C₈₋₁₂-Alkylrest bedeutet. Vorzugsweise ist der Rest R ein geradkettiger, gesättigter Alkylrest mit 8-10 Kohlenstoffatomen, besonders bevorzugt ist ein Alkylpolyglycosid mit einem Verhältnis von C₈- zu C₁₀-Alkylresten von 0,3:1 bis 1:0,3.

Der Rest ―(Z)ₙ―H ist ein Polyglycosylrest, d.h. ein einwertiger Rest aus n Glycosylresten, die vorzugsweise linear verknüpft sind, vorzugsweise ein reduzierender Saccharidrest. Der Polyglycosylrest kann ein Oligo- oder Monoglycosylrest sein. Beispiele als Alkylglycosylreste verwendbarer Zuckerkomponenten sind Glucose, Fructose, Mannose, Galactose, Talose, Gulose, Alose, Altrose, Idose, Arabinose, Ribose, Maltose, Laktose, Mutotriose, Dextrose, Stärke. Gemische dieser Zucker können ebenfalls eingesetzt werden. Bevorzugter Polyglycosylrest ist der Glucosylrest. Bevorzugt ist der Polyglycosylrest ―(Z)ₙ―H ein Oligosaccharid oder ein Monosaccharid, vorzugsweise einer Hexose oder Pentose, insbesondere Glucose.

Der Alkylrest kann in Form eines Acetals an mehr als einen Glucoserest gebunden sein, d.h. er kann an einen Poly- oder Oligosaccharidrest gebunden sein.

Die aliphatischen primären Alkohole können native Fettalkohole sein, die beispielsweise aus der Hydrierung von Fettsäuren hergestellt werden. Synthetische primäre Alkohole können ebenfalls eingesetzt werden, wie Oxoalkohole oder Ziegleralkohole. Bevorzugt werden geradkettige Alkohole eingesetzt, wie n-Octanol, n-Decanol, n-Dodecanol und deren Gemische.

Die Alkylpolyglycoside sind in der Regel Gemische, die neben geringen Mengen an Restalkohol auch nicht umgesetzte Monosaccharide, Oligosaccharide oder Oligoalkylpolyglycoside enthalten.

n beschreibt die mittlere Anzahl an Polyglykosylresten pro Alkoholrest und wird auch als Kondensationsgrad bezeichnet. n weist im Mittel einen Wert von 1 bis 5 auf, vorzugsweise von 1 bis 1,3.

Die erfindungsgemäß eingesetzten Alkylpolyglycoside können nach bekannten Verfahren hergestellt werden, wie sie beispielsweise in der DE OS-42 12 080, oder auch in den EP-A1-0 306 650, EP-A1-0 306 652, EP-A1-0 306 651 oder EP-A1-0 306 651 oder EP-A2-0 448 799 beschrieben sind. Sie können direkt durch Umsatz der langkettigen Alkoholkomponente mit der Zuckerkomponente unter Wasserabspaltung verknüpft werden. Sie können jedoch auch durch das Umacetalisierungsverfahren hergestellt werden, wobei zunächst ein kurzkettiges Alkylglycosid als Zwischenprodukt synthetisiert wird, das darauffolgend in einer zweiten Stufe durch Umacetalisierung mit langkettigen Alkoholen zum gewünschten Alkylpolyglycosid umgesetzt wird.

Die erfindungsgemäßen Ölemulsionen sind wahrscheinlich Mikroemulsionen von Öl in Wasser bzw. Wasser/Alkohol-Gemischen.

Besonders bevorzugt werden erfindungsgemäß die C₈/C₁₀-Alkylpolyglycoside, die gemäß einer Ausführungsform der Erfindung nach dem in der DE-OS-42 10 080 beschriebenen Verfahren hergestellt wurden, verwendet als Emulgiermittel für Öle, insbesondere etherische Öle.

Erfindungsgemäß einsetzbare Alkylpolyglycoside können auch Gemische von unterschiedlichen Alkylpolyglycosiden sein, die hergestellt werden durch Vermischen von Alkylpolyglycosiden mit Alkylmonoglycosiden.

### Coemulgatoren

Gemäß einer Ausführungsform der Erfindung können die Ölemulsionen zusätzlich Coemulgatoren enthalten. Diese Coemulgatoren sind beispielsweise nicht-ionische, kationische oder anionische Tenside. Bezogen auf den Alkylpolyglycosidanteil kann die Menge an eingesetztem Coemulgator 0 bis 15 Gew.-% betragen, vorzugsweise 0 bis 10 Gew.-%.

Beispiele verwendbarer Coemulgatoren sind Fettalkoholsulfate, Fettalko-holethersulfate, Alkansulfonate, Fettalkoholethoxilate, Fenalkoholphosphate, Fettalkoholethersulfonate, Alkylbetaine, Sorbitanester, POE-Sorbitanester, Zuckerfettsäureester, Fettsäurepolyglycerinester, Fettsäurepartialglyceride, Fettsäurecarboxilate, Fettalkoholsulfosuccinate, Fettsäuresarconisate, Fettsäureisethionate, Fettsäuretaurinate, Zitronensäureester, Silicon-Copolymere, Fettsäueepolyglykolester.

### Öl

In den erfindungsgemäßen Ölemulsionen können beliebige Öle verwendet werden. Vorzugsweise werden gemäß einer Ausführungsform der Erfindung etherische Öle verwendet. Beispiele für verwendbare etherische Öle sind Latschenkieferöl, Lavendelöl, Rosmarinöl, Fichtennadelöl, Kiefernnadelöl, Eukalyptusöl, Pfefferminzöl, Salbeiöl, Bergamottöl, Terpentinöl und deren Gemische. Andere geeignete etherische Öle sind dem Fachmann bekannt.

### Weitere Bestandteile

Die erfindungsgemäßen Ölemulsionen können weitere Bestandteile enthalten, beispielsweise Alkohole. Als Alkohol wird dabei vorzugsweise ein hautverträglicher, nicht-toxischer mit Wasser mischbarer Alkohol verwendet, wie Ethanol oder Isopropanol. Andere geeignete Alkohole können ebenfalls verwendet werden.

Die erfindungsgemäßen Ölemulsionen können auch frei von Alkohol sein.

Weitere Bestandteile, die den erfindungsgemäßen Ölemulsionen einverleibt werden können, sind beispielsweise Elektrolyte, Farbstoffe, Konservierungsmittel, Säuren, wie Milchsäure oder Zitronensäure, oder Basen.

### Herstellung der Ölemulsionen

Gemäß einer bevorzugten Ausführungsform der Erfindung wird das gewählte Alkylglycosid zunächst mit dem jeweils verwendeten etherischen Öl oder Parfümöl in den gewünschten Mengen innig gemischt, beispielsweise mittels eines Rührwerks. Dieses Vermischen wird vorzugsweise bei Raumtemperatur ausgeführt. Sodann wird unter ständigem Rühren langsam demineralisiertes Wasser eingetragen. Zum Auffinden der jeweils am besten geeigneten Mengen des Alkylglycosids zur Erzielung einer optimalen Solubilisierungswirkung kann die Trübungstitration verwendet werden, wie sie beispielsweise beschrieben ist von A. Domsch, "Die kosmetischen Präparate", 4. Auflage des von G.A. Novak begründeten Werks, Band II "Wäßrige und tensidhaltige Formulierungen".

Gemäß einer anderen Ausführungsform der Erfindung wird das Alkylglycosid zunächst in der wäßrigen Phase gelöst oder dispergiert und anschließend unter Rühren mit dem Öl versetzt.

Das Gewichtsverhältnis von Alkylglycosiden zu Öl oder Ölen beträgt dabei 1,5:1 bis 15:1, vorzugsweise 2,5:1 bis 10:1, insbesondere 3:1 bis 6:1. Das verwendete Alkylpolyglycosid, vorzugsweise das C₈/C₁₀-Alkylpolyglycosid, insbesondere das C₈/C₁₀-Alkylpolyglucosid, kann dabei als Festsubstanz oder als eine wäßrige Lösung eingesetzt werden. Gemäß einer Ausführungsform der Erfindung verwendete Mengen an Substanzen sind 3 bis 6 g (bezogen auf die Wirksubstanz) des gewünschten Alkylglycosids, 1 g des verwendeten Öls, vorzugsweise etherischen Öls oder Parfümöls, und demineralisiertes Wasser ad 100 g.

Die erfindungsgemäßen Ölemulsionen zeigen ein gutes Schaumvermögen, sind toxikologisch unbedenklich und klare und stabile Systeme.

Gemäß einer bevorzugten Ausführungsform werden C₈/C₁₀-Alkylpolyglycoside, insbesondere C₈/C₁₀-Alkylglucoside als Emulgatoren für etherische Öle eingesetzt.

### Bestimmung der Schaumhöhe

Zur Messung des Schaumvermögens und der Schaumstabilität wird die Schaumhöhe (Schaumzahl) für die erfindungsgemäßen Ölemulsionen bestimmt.

Dabei wird ein Gemisch aus Alkylpolyglycosid und Öl hergestellt und 1 g dieses Gemisches wird in einem 1 l fasssenden skalierten Becherglas mit hoher Form vorgelegt. Sodann werden 600 g demineralisiertes Wasser eingetragen. Daraufhin wird der Inhalt des Becherglases bei Raumtemperatur mittels eines elektrischen Rührwerks, bestehend aus einem IKA-Motor und einem Flügelrührer, der auf Höhe der 200 ml-Marke im Becherglas angeordnet ist, gemischt bzw. verrührt bei einer Drehzahl von etwa 1400 U/min.

Nach Rühren für eine Dauer von 1 Minute wird das Rührwerk abgestellt und nach 30 Sekunden die Schaumhöhe gemessen (in cm). Sodann wird das Rührwerk wieder gestartet und für weitere 3 Minuten bei gleicher Drehzahl gerührt. Nach wiederum 30 Sekunden Wartezeit wird die Schaumhöhe erneut gemessen (in cm). Die gemessenen Schaumhöhen (in cm) geben die jeweilige Schaumzahl an.

Statt Wasser kann auch ein Wasser/Alkohol-Gemisch eingesetzt werden, beispielsweise 600 g eines Wasser/Ethanol-Gemisches mit einem Gewichtsverhältnis von Wasser zu Ethanol von 80:20.

Nachstehend wird die Erfindung anhand von Beispielen näher erläutert.

Die Beispiele A1 bis A6 zeigen die Eigenschaften der erfindungsgemäßen Ölemulsionen. Die Emulsionen sind klar und über mehr als eine Woche stabil. Die Schaumwerte dieser Formulierungen liegen über denen der entsprechenden Vergleichsbeispiele (Vergleichsbeispiele B1 bis B4 zu Beispielen A1 bis A4). In den Vergleichsbeispielen B1 bis B6 wurde ein erfindungsgemäß verwendetes Alkylpolyglucosid eingesetzt, wobei das Gewichtsverhältnis von Alkylpolyglucosid zu etherischem Öl 1:1 betrug. Diese Formulierungen besitzen im Gegensatz zu den erfindungsgemäßen Formulierungen ein trübes bzw. milchiges Aussehen und sind nicht lange, d.h. weniger als 24 Stunden, stabil.

In Vergleichsbeispielen B7 bis B10 wurde ein handelsüblicher Solubilisator (ein ethoxiliertes hydriertes Rizinusöl mit der Bezeichnung Cremophor RH40) eingesetzt. Das Aussehen der Formulierungen ist trüb, und sie zeigen nur geringe Schaumwerte.

### Beispiel A1

1 g Rosmarinöl und 3 g C₈/C₁₀-Alkylpolyglucosid (Kondensationsgrad 1,2) werden mit 96 g Wasser vermischt und bei Raumtemperatur für 5 Minuten gerührt. Man erhält eine klare Lösung, die über einen langen Zeitraum, d.h. mehr als eine Woche, stabil ist.

### Beispiel A2

1 g Lavendelöl und 3 g C₈/C₁₀-Alkylpolyglucosid (Kondensationsgrad 1,2) werden mit 96 g Wasser vermischt und bei Raumtemperatur für 5 Minuten gerührt. Man erhält eine klare Lösung, die über einen langen Zeitraum, d.h. mehr als eine Woche, stabil ist.

### Beispiel A3

1 g Fichtennadelöl und 3 g C₈/C₁₀-Alkylpolyglucosid (Kondensationsgrad 1,2) werden mit 96 g Wasser vermischt und bei Raumtemperatur für 5 Minuten gerührt. Man erhält eine klare Lösung, die über einen langen Zeitraum, d.h. mehr als eine Woche, stabil ist.

### Beispiel A4

1 g Fichtennadelöl und 3 g C₈/C₁₀-Alkylpolyglucosid (Kondensationsgrad 1,2) werden mit 76,8 g Wasser und 19,2 g Ethanol vermischt und bei Raumtemperatur für 5 Minuten gerührt. Man erhält eine klare Lösung, die über einen langen Zeitraum, d.h. mehr als eine Woche, stabil ist.

### Beispiel A5

1 g Lavendelöl und 3 g C₈/C₁₀-Alkylpolyglucosid (Kondensationsgrad 1,2) werden mit 76,8 g Wasser und 19,2 g Ethanol vermischt und bei Raumtemperatur für 5 Minuten gerührt. Man erhält eine klare Lösung, die über einen langen Zeitraum, d.h. mehr als eine Woche, stabil ist.

### Beispiel A6

1 g des Mundpflegearomaöls Dragoco ZM0065 und 3 g C₈/C₁₀-Alkylpolyglucosid (Kondensationsgrad 1,2) werden mit 76,8 g Wasser und 19,2 g Ethanol vermischt und bei Raumtemperatur für 5 Minuten gerührt. Man erhält eine klare Lösung, die über einen langen Zeitraum, d.h. mehr als eine Woche, stabil ist.

Für die Lösungen gemäß Beispielen A1 bis A4 wurden die Schaumzahlen nach einer Minute und drei Minuten bestimmt. Sie sind in Tabellen 1 und 2 aufgeführt.

### Vergleichsbeispiele

### Beispiel B1

1 g Rosmarinöl und 1 g C₈/C₁₀-Alkylpolyglucosid (Kondensationsgrad 1,2) werden mit 98 g Wasser vermischt und bei Raumtemperatur für 5 Minuten gerührt. Man erhält eine stark trübe Lösung.

### Beispiel B2

1 g Lavendelöl und 1 g C₈/C₁₀-Alkylpolyglucosid (Kondensationsgrad 1,2) werden mit 98 g Wasser vermischt und bei Raumtemperatur für 5 Minuten gerührt. Man erhält eine milchige Flüssigkeit, die sich nach Stehenlassen für 24 Stunden in zwei Phasen trennt.

### Beispiel B3

1 g Fichtennadelöl und 1 g C₈/C₁₀-Alkylpolyglucosid (Kondensationsgrad 1,2) werden mit 98 g Wasser vermischt und bei Raumtemperatur für 5 Minuten gerührt. Man erhält eine milchige Flüssigkeit, die sich nach Stehenlassen für 24 Stunden in zwei Phasen trennt.

### Beispiel B4

1 g Fichtennadelöl und 1 g C₈/C₁₀-Alkylpolyglucosid (Kondensationsgrad 1,2) werden mit 78,4 g Wasser und 19,6 g Ethanol vermischt und bei Raumtemperatur für 5 Minuten gerührt. Man erhält eine stark trübe Lösung, die nach Stehenlassen für 24 Stunden milchig wird.

### Beispiel B5

1 g Lavendelöl und 1 g C₈/C₁₀-Alkylpolyglucosid (Kondensationsgrad 1,2) werden mit 78,4 g Wasser und 19,6 g Ethanol vermischt und bei Raumtemperatur für 5 Minuten gerührt. Man erhält eine milchige Lösung, die sich nach Stehenlassen für 24 Stunden in zwei Phasen trennt.

### Beispiel B6

1 g des Mundpflegearomaöls Dragoco ZM0065 und 1 g C₈/C₁₀-Alkylpolyglucosid (Kondensationsgrad 1,2) werden mit 78,4 g Wasser und 19,6 g Ethanol vermischt und bei Raumtemperatur für 5 Minuten gerührt. Man erhält eine milchige Lösung.

In den nachstehenden Vergleichsbeispielen B7 bis B10 wurde statt des C₈/C₁₀-Alkylpolyglucosids der handelsübliche Solubilisator Cremophor RH40 eingesetzt.

### Beispiel B7

1 g Rosmarinöl und 3 g Cremophor RH40 werden mit 96 g Wasser vermischt und bei Raumtemperatur für 5 Minuten gerührt. Man erhält eine stark trübe Lösung.

### Beispiel B8

1 g Rosmarinöl und 1 g Cremophor RH40 werden mit 98 g Wasser vermischt und bei Raumtemperatur für 5 Minuten gerührt. Man erhält eine stark trübe Lösung.

### Beispiel B9

1 g Fichtennadelöl und 3 g Cremophor RH40 werden mit 96 g Wasser vermischt und bei Raumtemperatur für 5 Minuten gerührt. Man erhält eine stark trübe Lösung.

### Beispiel B10

1 g Fichtennadelöl und 1 g Cremophor RH40 werden mit 98 g Wasser vermischt und bei Raumtemperatur für 5 Minuten gerührt. Man erhält eine stark trübe Lösung.

Für die Formulierungen der Vergleichsbeispiele B1 bis B4 und B7 bis B10 wurden die Schaumzahlen nach einer und drei Minuten bestimmt. Sie sind in Tabellen I und II aufgeführt.

**TABELLE I**

| **Schaumzahlen in Wasser** | | | | |
|---|---|---|---|---|
| **Beispiel** | **Emulgator bzw. Solubilisator** | **Eingesetztes Öl** | **Mischungsverhältnis** | **Schaumzahl 1 min./3 min.** |
| A1 | C₈-C₁₀ Glucosid | Rosmarinöl | 3(WS):1 | 2,2/4,2 |
| B1 | C₈-C₁₀ Glucosid | Rosmarinöl | 1(WS):1 | 1,9/3,8 |
| A2 | C₈-C₁₀ Glucosid | Lavendelöl | 3(WS):1 | 2,2/4,6 |
| B2 | C₈-C₁₀ Glucosid | Lavendelöl | 1(WS):1 | 1,6/2,9 |
| A3 | C₈-C₁₀ Glucosid | Fichtennadelöl | 3(WS):1 | 2,0/4,0 |
| B3 | C₈-C₁₀ Glucosid | Fichtennadelöl | 1(WS):1 | 1,4/2,0 |
| | | | | |
| B7 | Cremophor RH 40 | Rosmarinöl | 3(WS):1 | 1,2/2,1 |
| B8 | Cremophor RH 40 | Rosmarinöl | 1(WS):1 | 0,6/0,9 |
| B9 | Cremophor RH 40 | Fichtennadelöl | 3(WS):1 | 1,0/1,5 |
| B10 | Cremophor RH 40 | Fichtennadelöl | 1(WS):1 | 0,5/0,6 |

| **Schaumzahlen in Ethanol: Wasser - Gewichtsverhältnis 20:80** | | | | |
|---|---|---|---|---|
| **Beispiel** | **Emulgator** | **Eingesetztes Öl** | **Mischungsverhältnis** | **Schaumzahl 1 min./3 min.** |
| A4 | C₈-C₁₀ Glucosid | Fichtennadelöl | 3(WS):1 | 2,2/1,9 |
| B4 | C₈-C₁₀ Glucosid | Fichtennadelöl | 1(WS):1 | 1,5/2,0 |

Aus den Beispielen geht hervor, daß die erfindungsgemäßen Ölemulsionen klar sind und für lange Zeit stabil sind und ein gutes Schaumvermögen aufweisen. Die erfindungsgemäßen Alkylpolyglycoside können somit verwendet werden zur Herstellung von klaren Emulsionen von Öl in Wasser bzw. Wasser/Alkohol-Gemischen mit großer Stabilität und hohem Schaumvermögen. Im Gegensatz dazu weisen die Gemische entsprechend den Vergleichsbeispielen ein schlechteres Schaumvermögen auf. Sie sind trübe oder milchige Flüssigkeiten, die sich zum Teil in zwei Phasen trennen.

Die erfindungsgemäßen Emulsionen können beispielsweise in der Kosmetik oder in pharmazeutischen Anwendungen eingesetzt werden, beispielsweise in Badeölen, Rasierwässern, Gesichtswässern, Mundwässern, Haarwässern. Ölemulsionen, die Alkohol enthalten, können als Eau de Cologne oder Fau de Toilette verwendet werden.

## Patentansprüche

1. Ölemulsion, umfassend
(a) mindestens ein Alkylpolyglycosid der allgemeinen Formel
R―O―(Z)ₙ―H
in der der Rest R ein linearer oder verzweigter gesättigter oder ungesättigter Alkylrest mit 8 bis 16, vorzugsweise 8 bis 12, C-Atomen ist, der Rest ―(Z)ₙ―H ein Polyglycosylrest ist, Z ein Glycosylrest ist und n im Mittel einen Wen von 1 bis 5 aufweist,
(b) mindestens ein Öl,
(c) Wasser,
wobei das Gewichtsverhältnis von Komponente (a) zu Komponente (b) 1,5:1 bis 15:1, vorzugsweise 2,5:1 bis 10:1, beträgt.

2. Ölemulsion nach Anspruch 1, die weiterhin (d) mindestens einen mit Wasser mischbaren Alkohol umfaßt.

3. Ölemulsion nach Anspruch 1, die frei von Alkohol ist.

4. Ölemulsion nach einem der vorstehenden Ansprüche, wobei das Alkylpolyglycosid ein C₈/C₁₀-Alkylpolyglycosid ist.

5. Ölemulsion nach einem der vorstehenden Ansprüche, wobei der Polyglycosylrest ―(Z)ₙ―H, ein Oligosaccharid oder ein Monosaccharid, vorzugsweise einer Hexose oder Pentose, insbesondere Glucose, ist.

6. Ölemulsion nach einem der vorstehenden Ansprüche, wobei das Öl ein etherisches Öl ist.

7. Ölemulsion nach einem der vorstehenden Ansprüche, die zusätzlich (e) mindestens einen Coemulgator umfaßt, der ein nicht-ionisches, kationisches oder anionisches Tensid ist.

8. Verwendung von Alkylpolyglycosiden, wie sie in Anspruch 4 oder einem der auf Anspruch 4 bezogenen Ansprüche beschrieben sind, als Emulgiermittel für Öle, insbesondere etherische Öle.

9. Verwendung von Alkylpolyglycosiden, wie sie in einem der vorstehenden Ansprüche beschrieben sind, zur Herstellung von klaren Emulsionen von Öl in Wasser bzw. Wasser/Alkohol-Gemischen.

10. Verwendung der Ölemulsion nach einem der Ansprüche 1 bis 7 in Badeöl, Rasierwasser, Gesichtswasser, Mundwasser oder Haarwasser.
